**Europäisches Patentamt**

**European Patent Office**

**Office européen des brevets**

(11) Veröffentlichungsnummer: **0 513 419 A1**

# (12) EUROPÄISCHE PATENTANMELDUNG

(21) Anmeldenummer: **91107989.5**

(22) Anmeldetag: **16.05.91**

(51) Int. Cl.5: **A61B 5/16, G09B 5/04**

(43) Veröffentlichungstag der Anmeldung:
**19.11.92 Patentblatt 92/47**

(84) Benannte Vertragsstaaten:
**DE FR GB**

(71) Anmelder: **Ludwig, Lothar, Dr.**
**Jägerluststrasse 5**
**W-8347 Kirchdorf(DE)**
Anmelder: **Pigache, Robert, Dr.**
**43a, boulevard Clemenceau**
**F-67000 Strasbourg(FR)**

(72) Erfinder: **Ludwig, Lothar, Dr.**
**Jägerluststrasse 5**
**W-8347 Kirchdorf(DE)**
Erfinder: **Pigache, Robert, Dr.**
**43a, boulevard Clemenceau**
**F-67000 Strasbourg(FR)**

(74) Vertreter: **Klunker . Schmitt-Nilson . Hirsch**
**Winzererstrasse 106**
**W-8000 München 40(DE)**

(54) **Gerät zum Testen der Aufmerksamkeit einer Person.**

(57) Bei einem Gerät zum Testen der Aufmerksamkeit oder Konzentrationsfähigkeit einer Person werden aus einem Sprachspeicher (62) mit einer vorbestimmten, änderbaren Geschwindigkeit Ziffernfolgen ausgelesen und über einen Sprachgenerator (66) auf einem Kopfhörer (10) geleitet. Bei jedem Auftreten einer "0" muß die Testperson einen Taster (16) betätigen. Auch wird bei jedem Auftreten einer "0" von einer Steuer-Einheit (50) ein Fenstersignal erzeugt, welches von einer Fehlerlogikschaltung (70) mit dem Tasterimpuls verknüpft wird. Bei korrekter Reaktion liegt der Tasterimpuls im Zeitfenster, und wird der Stand des Zählers (74) für die "Treffer" um eins erhöht. Die Differenz zwischen der bekannten Anzahl möglicher Nullen und dem Zählerstand dieses Zählers gibt die Anzahl von Auslassungsfehlern an. Außerhalb der Zeitfenster anfallende Tasterimpulse sind Einfügungsfehler, d. h. Tastenbetätigung ohne vorausgegangene "0". Diese werden in einem weiteren Zähler (75) gezählt. Die Zählerstände werden dann zur Errechnung eines Fehlerindex ($I_E$) verarbeitet.

Fig. 1

EP 0 513 419 A1

Die Erfindung betrifft ein Gerät zum Testen der Aufmerksamkeit einer Person.

Es gibt für verschiedene Zwecke bestimmte Geräte, um die Aufmerksamkeit oder Konzentrationsfähigkeit einer Person zu testen. Ein derartiger Test wurde z. B. von R. M. Pigache für an Schizophrenie leidende Patienten vorgeschlagen (Perceptual and Motor Skills, 1976, 42, 243-253).

Nach dem genannten Vorschlag werden einer Testperson Zahlenreihen zu Gehör gebracht, und die Testperson muß auf bestimmte Zahlen, z. B. die Zahl "0", reagieren, indem z. B. eine Hand- oder Fußtaste betätigt wird. Dieser Test kann so durchgeführt werden, daß in einer ersten Phase die Testperson auf beiden Ohren die gleichen Reizworte (Stimuli) hört, in einer nachfolgenden Testphase die Reizworte in unterschiedlicher Reihenfolge auf verschiedenen Ohren hört, jedoch nur auf bestimmte Zielworte (Targets), im folgenden Beispiel also die "0", reagieren muß, während die Information am anderen Ohr lediglich als Störsignal dient. Die Zahlenreihen können mit unterschiedlichen Geschwindigkeiten der Testperson zu Gehör gebracht werden.

Nun ist aber die Durchführung des oben skizzierten Tests mit erheblichem Personalaufwand verbunden. Wenn beispielsweise eine Krankenschwester oder medizinischtechnische Assistentin der Testperson die Zahlenreihen vorliest, ist eine besondere Aufmerksamkeit seitens des Personals erforderlich, um einerseits die Zahlenreihen mit gleichmäßiger Geschwindigkeit und deutlicher Aussprache vorzulesen, während andererseits exakt beobachtet werden muß, ob die Testperson auf die Targets richtig reagiert bzw. fälschlicherweise auf nicht vorhandene Targets reagiert. Vor der Durchführung des Tests muß die Testperson instruiert werden.

Die oben aufgezeigten Umstände machen das Testergebnis insoweit fragwürdig, als die Reproduzierbarkeit sämtlicher Testbedingungen nicht gewährleistet ist.

Der Erfindung liegt die Aufgabe zugrunde, ein Gerät zum Testen der Aufmerksamkeit einer Person anzugeben, welches eine exakte Reproduzierbarkeit der einzelnen Tests gewährleistet und außerdem bei geringem Personalaufwand exakte Meßergebnisse liefert.

Demgemäß schlägt die Erfindung folgende Merkmale für das Testgerät vor:
- aus einem Sprachspeicher werden in gleichmäßigen Zeitabständen Stimuli (Reizworte) ausgelesen;
- die Stimuli werden mittels eines Sprachgenerators über Lautsprecher oder Kopfhörer für die Testperson hörbar gemacht,
- ein von der Testperson bei Erkennen eines bestimmten Stimulus-Typs (Target) zu betätigender Taster liefert Tasterimpulse, und
- eine Registriereinrichtung registriert die Tasterimpulse und korreliert diese sowohl mit den Targets als auch mit den von den Targets verschiedenen Stimuli.

Es hat sich als zweckmäßig erwiesen, als Stimuli Ziffern zu verwenden, wobei jeweils die Ziffer "0" als Target dient.

Mit dem in oben beschriebener Weise ausgestatteten Gerät lassen sich bei verschiedenen Testpersonen und/oder zu verschiedenen Test-Zeitpunkten sämtliche Testbedingungen genau reproduzieren. Dies gewährleistet aussagekräftige Meßergebnisse. In dem Sprachspeicher sind die Reizwort in einer Pseudo-Zufallsfolge gespeichert, und sie werden durch Auslesen des Sprachspeichers mit einem bestimmten Betriebstakt in bestimmten Zeitabständen ausgelesen und hörbar gemacht. Die Testperson betätigt bei jedem Erkennen einer "0" den Taster und dieser Taster liefert einen Tasterimpuls zu der Registriereinrichtung. In der Registriereinrichtung werden neben den vom Taster kommenden Tasterimpulsen auch die Informationen über die Stimuli und die Targets registriert. Damit ergibt sich für die Auswertung der einzelnen Tests die günstige Situation, durch Vergleich sehr schnell zu erkennen, ob die Testperson korrekt auf die einzelnen Targets reagiert hat und/oder ob die Testperson den Taster betätigt hat, obschon kein Target gehört wurde.

Zur Erhöhung der Reproduzierbarkeit der Testbedingungen trägt noch bei, daß ein Informationssprachspeicher und/oder eine Anzeige vorgesehen sind als Mittel, der Testperson die zur Durchführung des Tests benötigten Informationen mitzuteilen. Es hat sich nämlich erwiesen, daß das Instruieren einer Testperson durch beispielsweise einer medizinisch-technischen Assistentin im Einzelfall zwar ausreicht, daß diese Instruktionen sich aber von Fall zu Fall unterscheiden, was sich auf die Meßergebnisse auswirkt.

Wie oben angedeutet, besteht ein ganz einfacher Test darin, daß über einen Kopfhörer beiden Ohren einer Testperson identische Ziffernfolgen zugeführt werden, so daß die Testperson jedesmal reagieren muß, wenn sie eine "0" hört. Das Auslesen der Ziffernfolgen aus dem Sprachspeicher und das Generieren der akustischen Information durch den Sprachgenerator erfolgt mit einer bestimmten Taktgeschwindigkeit. Diese Taktgeschwindigkeit läßt sich ändern. Vorzugsweise sind zwei verschiedene Lesegeschwindigkeiten für den Sprachspeicher bzw. Sprechgeschwindigkeiten für die Stimuli vorgesehen: In einer schnellen Betriebsart hört die Testperson pro Sekunde zwei Reizwerte, in einer langsamen Betriebsart hört die Testperson alle zwei Skunden ein Reizwort.

Die oben beschriebene Betriebsart läßt sich als "Mono-Betriebsart" bezeichnen. Es werden "diotische Stimuli" bereitgestellt.

Um aussagekräftigere Meßergebnisse zu erhalten, sieht die Erfindung außerdem vor, daß zusätzlich und alternativ sogenannte dichotische Stimuli erzeugt werden. Das heißt, es werden über getrennte Kanäle dem linken und dem rechten Ohr der Testperson unterschiedliche Folgen von Stimuli zugeleitet. Die Testperson reagiert lediglich auf Targets, die er mit dem einen Ohr wahrnimmt, während die Stimuli am anderen Ohr lediglich Störgeräusche darstellen. Dabei wird dafür Sorge getragen, daß nicht gleichzeitig ein Target auf beiden Ohren zu hören ist.

Erfindungsgemäß wird diese Variante des Tests dadurch realisiert, daß der Sprachgenerator mit zwei Kanälen für ein Kopfhörerpaar ausgestattet ist, daß eine Steuereinrichtung eine Umschalteinrichtung aufweist, mit der entweder gleiche Stimuli gleichzeitig auf beiden Kanälen oder verschiedene Stimuli auf den Kanälen erzeugt werden.

Kombiniert man diese Merkmale mit einer Geschwindigkeitsumschalteinrichtung, mit deren Hilfe eine von mehreren Auslesegeschwindigkeiten der Stimuli auswählbar ist, so ergeben sich grundsätzlich vier verschiedene Testarten: Erstens, diotische Stimuli bei langsamer Geschwindigkeit; zweitens, dichotische Stimuli bei langsamer Geschwindigkeit; drittens, diotische Stimuli bei hoher Geschwindigkeit; viertens, dichotische Stimuli bei hoher Geschwindigkeit.

Mit dem erfindungsgemäßen Gerät lassen sich dann beispielsweise nacheinander vier Unter- oder Subtests durchführen, von denen jeder beispielsweise fünf Minuten dauert. In der ersten 5-Minuten-Phase werden dichotische Stimuli bei schneller Geschwindigkeit der Testperson zugeleitet. In den fünf Minuten ( = 300 Sekunden) hört die Testperson also auf beiden Ohren gleichzeitig insgesamt 600 Ziffern, darunter sind 50 Nullen.

Das beste Ergebnis besteht also darin, daß die Testperson unmittelbar nach dem Hören jeder Null den Taster betätigt, ohne bei den übrigen 550 Nicht-Nullen auch nur ein einziges Mal den Taster zu betätigen. Versäumt es die Testperson, als Reaktion auf eine gehörte "Null" den Taster zu betätigen, so entsteht ein Fehler, der hier als Auslassungsfehler (omission error) bezeichnet werden soll. Reagiert die Testperson falsch, d. h. betätigt sie den Taster, ohne daß sie eine "Null" hört, so entsteht ein hier als Einfügungsfehler (commission error) bezeichneter Fehler. Ab nun muß aber ein Kriterium definiert werden, gemäß welchem beurteilt wird, ob die Betätigung des Tasters durch die Testperson als Reaktion auf eine gehörte "Null" erfolgt ist oder beispielsweise irrtümlich. Deshalb ist bei dem erfindungsgemäßen Gerät vorgesehen, daß eine Zeitsteuereinrichtung ein Fenstersignal vorbestimmter Zeitdauer bei jeder Ausgabe eines Targets erzeugt, und daß eine Verknüpfungsschaltung die Fenstersignale mit den Tasterimpulsen verknüpft. Wenn die Ziffernfolge langsam gesprochen wird, z. B. mit einer Geschwindigkeit von einer Ziffer pro zwei Sekunden, beträgt die Zeitspanne des Fenstersignals beispielsweise vier Sekunden. Bei schneller Folge der Ziffern beträgt die Zeitspanne des Fenstersignals nur 1,5 Skunden. Wenn die zu testende Person nach dem Hören einer "Null" solange mit dem Betätigen des Tasters wartet, bis das Fenstersignal wieder verschwunden ist, wird die Betätigung des Tasters als Einfügungsfehler gewertet.

Das erfindungsgemäße Gerät läßt sich in seiner einfachsten Ausführungsform mit Hilfe eines Mehrspur-Tonbandgeräts realisieren. Dabei wird auf einer ersten Spur ein Taktsignal, auf der zweiten Spur eine Ziffernfolge für beispielsweise den linken Kanal, auf der dritten Spur eine Ziffernfolge für den rechten Kanal und auf der vierten Spur eine Impulsfolge, die das Auftreten jeweils einer "Null" kennzeichnet, gespeichert. Die Registriereinrichtung kann dann z. B. ein Mehrkanal-Schreiber (Polygraph) sein, wobei in einer ersten Spur die Taktsignale und die genannten Target-Impulse aufgezeichnet werden, während in einer weiteren Spur die Tasterimpulse aufgezeichnet werden. Die Auswertung der Aufzeichnung kann dann "manuell" oder mittels eines geeigneten Lesegeräts erfolgen. Es wird festgestellt, ob die einzelnen Tasterimpulse jeweils einen Höchstabstand von einem vorausgehenden Target-Impuls aufweisen. So lassen sich Auslassungsfehler und Einfügungsfehler feststellen. Die Auswertung erfolgt dann mit einem üblichen Rechner.

Zum Ermitteln der Auslassungfehler ist ein erster Zähler vorgesehen, der jeden Tasterimpuls zählt, der mit einem Fenstersignal zusammenfällt. Da die Anzahl der Targets oder Nullen in einer Ziffernfolge bekannt ist, läßt sich die Anzahl der Auslassungsfehler durch Subtrahieren des Zählerstands von der Anzahl der Nullen ermitteln.

In einem zweiten Zähler werden Tasterimpulse gezählt, die nicht mit einem Fenstersignal zusammenfallen. Bei einer gegebenen Anzahl von Stimuli und gegebene Anzahl von Targets errechnet sich als Differenz aus diesen Werten die Anzahl der Nicht-Targets. Dazu im Verhältnis steht der Inhalt des Zählerstands des zweiten Zählers, kennzeichnend für die Einfügungsfehler.

Vorzugsweise wird das erfindungsgemäße Gerät als praktisch vollständig digital arbeitendes Gerät ausgebildet. In einem Schreib/Lese-Speicher (RAM) werden in digitalisierter Form Sprachsignale für die einzelnen Ziffern 0 bis 9 gespeichert. In einem weiteren Speicher werden Adreßsignale für den Sprachspeicher gespeichert, und der Adreßspeicher seinerseits wird von einem Adreßregister kontinuierlich adressiert. In dem Adreßspeicher sind die Adressen für den Sprachspeicher vorab auf Pseudo-Zufallsbasis gespeichert worden. Für eine 2-Kanal-Anlage sind zwei Adreßspeicher und Sprachspeicher und auch zwei Sprachgeneratoren vorgesehen.

Die Auswertung erfolgt durch eine Rechenschaltung, die aus den registrierten Tasterimpulsen einen Fehlerindex und davon abgeleitete Werte berechnet.

Eine bevorzugte Formel für den Fehlerindex $I_E$ lautet:

(1) $I_E = O_m/n_t + 2 \cdot Co/(S-n_t)$

$I_E$ = Fehlerindex
$O_m$ = Auslassungsfehler
$n_t$ = Anzahl der Targets
$C_o$ = Einfügungsfehler
$S$ = Gesamtzahl der Stimuli incl. $n_t$

Die Auslassungsfehler und Einfügungsfehler errechnen sich anhand der erwähnten Zählerstände wie folgt:

(2a) $O_m = n_t - \langle$Zähler 1$\rangle$

(2b) $Co = \langle$Zähler 2$\rangle$

Man erhält dann beispielsweise für einen Subtest, bei dem 600 dichotische Stimuli mit 50 Targets in schneller Aufeinanderfolge gelesen werden, folgenden Fehlerindex:

$$(3) \quad I_E(F_2) = \frac{(50 - 35)}{50} + 2 \cdot \frac{80}{600 - 50}$$
$$= 0,58$$

Man kann daran denken, die oben erwähnten vier jeweils fünf Minuten dauernden Einzeltests (schnell/langsam für jeweils diotische und dichotische Stimuli) zusammenzufassen, um sämtliche Ergebnisse gemeinsam auszuwerten, beispielsweise den oben angegebenen Fehlerindex zu berechnen.

Aussagekräftiger ist jedoch eine separate Auswertung jedes einzelnen Subtests und die Verknüpfung der für jeden Subtest ermittelten Fehlerindizes $I_E$ mit den Ergebnissen der anderen Subtests.

Die aus dem Sprachspeicher jeweils auf Pseudo-Zufallsbasis ausgelesenen Sprachsignale werden von einem hier als Sprachgenerator bezeichneten Schaltungsteil weiterverarbeitet, insbesondere demoduliert (bei deltamodulierten gespeicherten Sprachsignalen) und verstärkt, um über die Kopfhörer eines Kopfhörerpaares ausgegeben zu werden.

Der gesamte Testablauf, welcher mehrere Subtests umfaßt, wird automatisch dadurch gesteuert, daß eine Steuereinheit ein Zeitzählwerk steuert und automatisch eine Reihe von Subtests mit jeweils voneinander verschiedenen Parametern (diotische/dichotische Stimuli; schnell/langsam) ablaufen läßt.

Die Rechenschaltung verarbeitet die bei jedem der Subtests registrierten Tasterimpulse einzeln, und die Verarbeitungsergebnisse werden anschließend miteinander verknüpft.

Für die digitale Version des erfindungsgemäßen Geräts ist vorgesehen, daß der Sprachspeicher ein digitaler Speicher (z. B. RAM) ist, in welchem den einzelnen Stimuli entsprechende Sprachsignale unter jeweils einer Adresse oder Adressengruppe gespeichert sind, daß ein Adressenspeicher mit seinem Ausgang an den Adreßeingang des Sprachspeichers angeschlossen ist und daß in dem über ein Adreßregister schrittweise adressierten Adressenspeicher Sprachspeicheradressen auf Pseudo-Zufallsbasis gespeichert sind.

Für den Wechsel zwischen langsamer und schneller Ziffernfolge ist ein Taktgeber mit umschaltbarer Taktfrequenz vorhanden, an welchem das Adreßregister angeschlossen ist.

Im folgenden werden Ausführungsbeispiele der Erfindung anhand der Zeichnungen näher erläutert. Es zeigen:

Fig. 1     ein Blockdiagramm eines Geräts zum Testen der Aufmerksamkeit und der Konzentrationsfähigkeit einer Person,

Fig. 2     eine schematische Darstellung einer Einrichtung zum Erzeugen von gesprochenen Ziffernfol-

EP 0 513 419 A1

gen,

Fig. 3     eine Skizze einer Registriereinrichtung in Form eines Polygraphen,

Fig. 4     ein Blockdiagramm einer digitalen Ausführungsform des erfindungsgemäßen Geräts,

Fig. 5     ein detailliertes Schaltbild der in Fig. 4 als Block dargestellten Fehlerlogik,

Fig. 6     eine Skizze, die den Zusammenhang zwischen gesprochenen Ziffernfolgen und der Erfassung von verschiedenen Fehlertypen veranschaulicht.

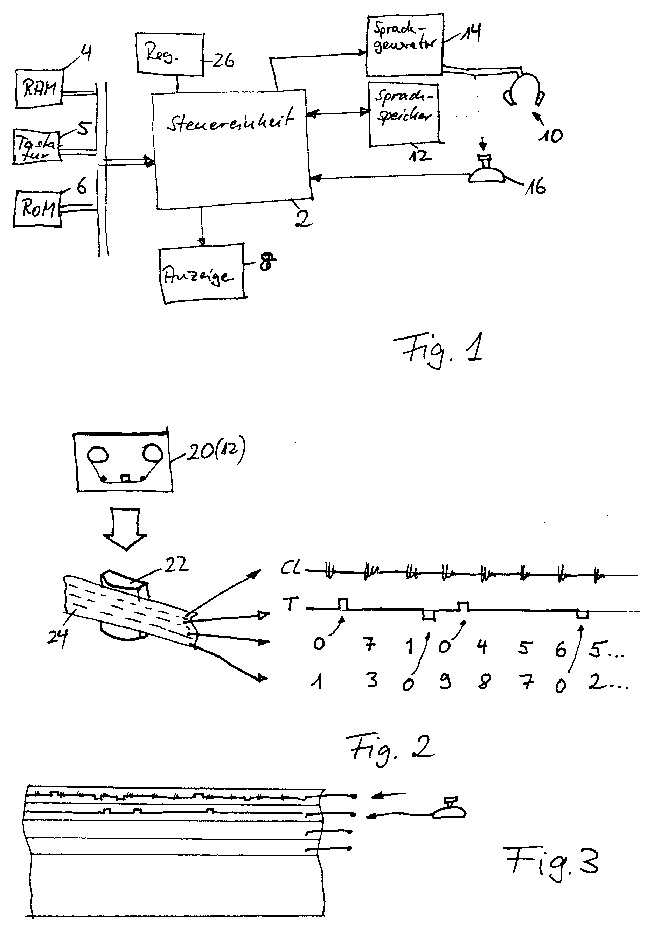

Das in Fig. 1 dargestellte Blockschaltbild zeigt eine Steuereinheit, die einen Mikroprozessor, einen Mikrocomputer od. dgl. enthält. Angeschlossen an die Steuereinheit 2 sind über einen links in Fig. 1 dargestellten Bus ein Schreib/Lese-Speicher 4, eine Tastatur 5 und ein Festspeicher (ROM) 6. In den beiden Speichern 4 und 6 sind Programme, Daten u. dgl. für den Betrieb der Steuereinheit 2 gespeichert. Über die Tastatur 5 können Befehle an die Steuereinheit 2 gegeben werden. Eine Anzeige 8 dient zur Anzeige von Testergebnissen, aber auch zur Anzeige von Instruktionen für die Testperson in Vorbereitung eines Tests.

An die Steuereinheit 2 angeschlossen ist ferner ein Sprachspeicher 12, in welchem Ziffernfolgen gespeichert sind. Ein Sprachgenerator 14 verarbeitet die Signale aus dem Sprachspeicher 12, um sie in NF-Signale für zwei Kanäle umzusetzen, die auf die beiden Kopfhörer eines Kopfhörerpaares 10 gegeben werden.

Beim einfachsten Beispiel ist der Sprachspeicher 12 beispielsweise als Mehrspur-Tonbandkassette ausgebildet.

Fig. 2 zeigt schematisch eine Tonbandkassette 20, einen Mehrspur-Tonkopf 22 und ein daran entlanggeführtes Vierspur-Tonband 24. Auf der obersten Spur ist ein Taktsignal Cl in Form von Hochfrequenz-Signalburst gespeichert.

Auf der zweiten Spur sind sogenannte Target-Impulse gespeichert, die angeben, wo in der dritten bzw. in der vierten Spur jeweils eine "0" gespeichert ist. Entsprechend ist die Polarität der einzelnen Target-Impulse T.

In der dritten Spur ist hier die Ziffernfolge 0, 7, 1... gespeichert, während in der vierten Spur die Ziffernfolge 1, 3, 0, 9 ... gespeichert ist.

Die in Fig. 1 dargestellte Steuereinheit 2 steuert den Betrieb des Tonbands, d. h. des Sprachspeichers 12. Über den Sprachgenerator 14 (der z. B. einen Verstärker enthält) werden die Ziffernfolge über die beiden Kanäle des Kopfhörers 10 gegeben. Die den Kopfhörer 10 tragende Testperson hört auf dem linken Ohr beispielsweise die Ziffernfolge 0, 7, 1 ... von der dritten Spur, und auf dem rechten Ohr die Ziffernfolge 1, 3, 0, 9 ... von der vierten Spur. Gemäß den vorabgegebenen Instruktionen ist dann z. B. lediglich die auf dem linken Ohr gehörte Ziffernfolge 0, 1, 2, 0 ... "relevant", während die auf dem anderen Ohr gehörte Ziffernfolge lediglich als Störung dient. Jedesmal, wenn die Testperson eine "0" auf dem linken Ohr hört, muß sie einen Taster 16 betätigen, welcher dann einen Tasterimpuls an die Steuereinheit 2 liefert. Die Steuereinheit 2 gibt dann entsprechende Signalimpulse an eine Registriereinheit 26.

Die Registriereinheit 26 kann beispielsweise ein Polygraph sein, wie er in Fig. 3 dargestellt ist. Während in dem oberen Kanal gemeinsam die Taktsignale Cl und die Target-Impulse T gespeichert werden, werden im zweiten Kanal die Tasterimpulse aufgezeichnet. Durch automatische oder "manuelle" Auswertung läßt sich feststellen, ob jeweils auf den für den Kanal relevanten Target-Impuls (Polarität!) innerhalb eines bestimmten Zeitraums ein Tasterimpuls vorhanden ist. Daraus läßt sich die Anzahl von Auslassungfehlern ebenso feststellen wie die Anzahl von Einfügungsfehlern.

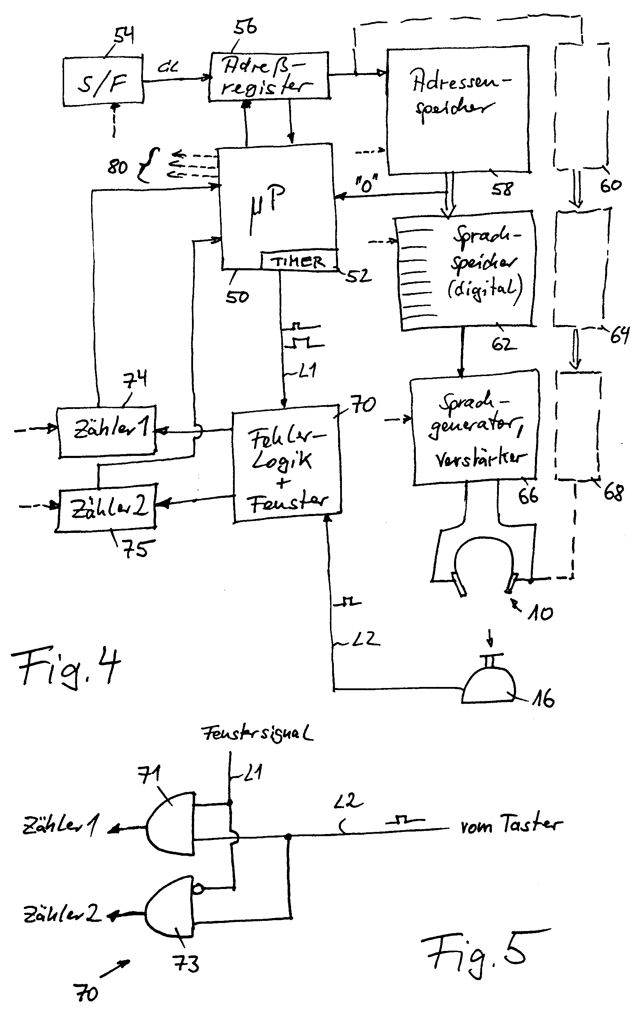

Eine in vieler Hinsicht komfortablere Ausgestaltung der in Fig. 1 grob skizzierten Anlage ist in Fig. 4 dargestellt. Die gesamte Anlage arbeitet digital.

Ein zur Steuerung dienender Mikroprozessor 50 mit einer Zeitgeberschaltung (TIMER) 52 steuert verschiedene Schaltungsteile, die im folgenden näher beschrieben werden. Die Steuerung erfolgt anhand eines Betriebsprogramms, welches beispielsweise in dem in Fig. 1 gezeigten (in Fig. 4 fortgelassenen) ROM 6 und/oder RAM 4 gespeichert ist.

In Fig. 4 sind an einzelnen Blöcken gestrichelte Steuersignal-Enden dargestellt. Diese kommen von den Steuersignal-Ausgängen 80 des Mikroprozessors 50.

Ein auf eine hohe und eine niedrige Taktfrequenz schaltbarer Taktgeber 54 liefert Taktimpulse Cl an das Adreßregister 56. Wenn dieses von dem Mikrocomputer 50 gestartet wird, adressiert es fortlaufend Speicherplätze eines Adressenspeichers 58. In dem Adressenspeicher 58 sind vorab auf PseudoZufallsbasis Adressen eines digitalen Sprachspeichers 62 gespeichert. Die Sprachsignale (Ziffern) sind deltamoduliert gespeichert, und jeweils für eine Ziffer wird ein Bereich oder eine Adresse bzw. Adressengruppe aus dem Sprachspeicher ausgelesen, wobei diese Adressengruppe jeweils vom Ausgangssignal des Adressenspeichers 58 festgelegt wird. Wird eine "0" ausgelesen, so wird dies dem Mikroprozessor 50 signalisiert, der sofort über den Timer 52 ein Fenstersignal auf eine Leitung L1 gibt. Abhängig davon, ob die Ziffernfolge schnell oder langsam ausgelesen wird (Frequenz des Taktsignals Cl), dauert das Fenstersignal vier Sekunden bzw. 1,5 Sekunden.

Von dem Sprachspeicher gelangen die Signale für jeweils eine Ziffer zu einem Sprachgenerator 66, der beispielsweise eine Demodulatorschaltung und einen Verstärker enthält. Für den Monobetrieb (diotische Stimuli) werden auf beide Kopfhörer eines Kopfhörerpaares 10 dann die einzelnen Ziffern gegeben. Die Testperson hört die Ziffernfolge und betätigt bei jeder "0" den Taster 16, so daß über eine Leitung L2 ein Tasterimpuls an eine Fehlerlogik- und Fensterschaltung 70 gegeben wird. Letztere Schaltung empfängt auch von dem Mikrocomputer 50 über die Leitung L1 das Fenstersignal.

Fig. 5 zeigt eine mögliche Hardware-Ausgestaltung der Schaltung 70. Das Fenstersignal wird zusammen mit dem Tastersignal auf ein UND-Glied 71 gelegt. Außerdem gelangt an ein weiteres UND-Glied 73 das invertierte Fenstersignal von der Leitung L1, sowie von der Leitung L2 das Tastersignal.

Gemäß Fig. 4 ist an das UND-Glied 71 ein Zähler 1 angeschlossen, während an das UND-Glied 73 ein Zähler 2 angeschlossen ist.

Wenn nun die Testperson rasch genug reagiert, kommt der aufgrund einer gehörten "0" erzeugte Tasterimpuls über die Leitung L2 am UND-Glied 71 an, solange das Fenstersignal erzeugt wird. Dadurch entsteht am Ausgang des UND-Glieds 71 ein Impuls, durch den der Zählerstand des Zählers 74 (Zähler 1) um eins erhöht wird.

Bei bekannter Anzahl von Nullen pro Testfolge läßt sich dann die Anzahl der Auslassungsfehler durch Subtrahieren des Zählerstands des Zählers 74 von der Anzahl der Targets ermitteln.

Der Inhalt des Zählers 75 (Zähler 2) entspricht der Anzahl von Tasterimpulsen, die außerhalb sämtlicher Fenstersignale erzeugt wurden. Dies entspricht der Anzahl von Einfügungsfehlern.

Durch Umschalten der Frequenz des Taktsignals Cl des Taktgebers 54 lassen sich schnelle und langsame Ziffernfolgen (Stimuli) erzeugen.

Eine weitere Variante wird dadurch erhalten, daß auf die beiden Kopfhörer des Kopfhörerpaares 10 verschiedene Ziffernfolgen gegeben werden, wie es schematisch in Fig. 2 dargestellt ist. Hierzu sind in der Ausführungsform nach Fig. 4 zusätzliche Bausteine vorhanden, nämlich ein zusätzlicher Adressenspeicher 60, ein zusätzlicher Sprachspeicher 64 und ein zusätzlicher Sprachgenerator 68.

Die Auswertung der Zählerstände der Zähler 74 und 75 mit den gespeicherten Zahlenwerten für die Gesamtzahl der Stimuli und die Gesamtzahl der Targets erfolgt innerhalb des Mikroprozessors gemäß einem bestimmten Auswertungsprogramm. So erfolgt für jeden einzelnen Subtest, der jeweils fünf Minuten dauert, die Berechnung des Fehlerindex $I_E$ (Formel 1), und ausgehend von den einzelnen Fehlerindizes $I_E$ für die einzelnen Subtests erfolgt eine weitere Verknüpfung, um eine Aussage über die Konzentrationsfähigkeit bzw. die Aufmerksamkeit der Testperson zu erhalten.

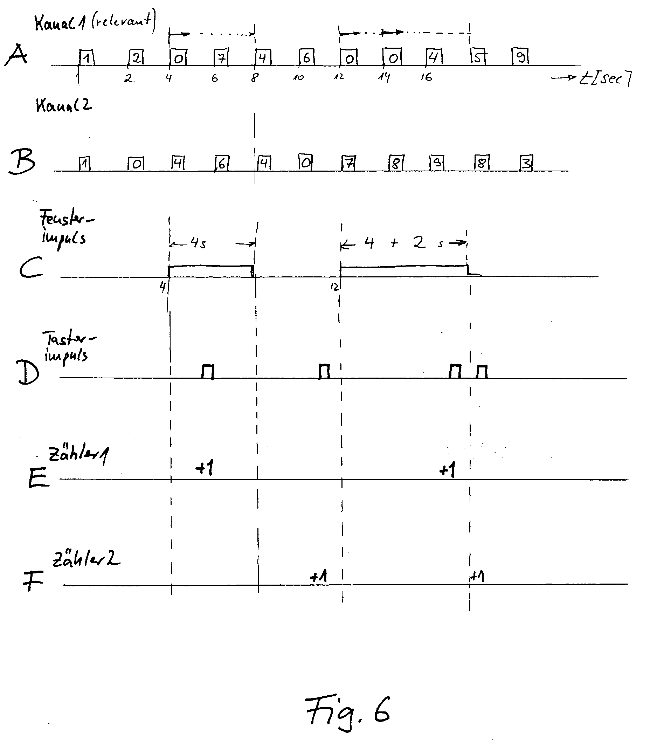

Fig. 6 zeigt anhand eines Impulsdiagramms ein Beispiel für das Erfassen der Fehler.

In Fig. 6A und Fig. 6B sind die gesprochenen Ziffernfolgen für jeweils einen Kanal 1 bzw. einen Kanal 2 dargestellt. Beispielsweise muß die Testperson die über den Kanal 1 auf das linke Ohr gegebene Ziffernfolge beachten, während die auf das rechte Ohr gegebene Ziffernfolge des Kanals 2 ignoriert werden muß. Die Ziffernfolgen sind so beschaffen, daß maximal drei Nullen nacheinander auftauchen, wobei in den beiden Kanälen nie gleichzeitig eine Null auftritt.

Fig. 6A und 6B veranschaulichen die Ziffernfolge für einen "Langsam-Test" mit dichotischen Stimuli (verschiedene Ziffernfolgen auf beiden Ohren). Alle zwei Sekunden wird eine Ziffer gesprochen. Jedesmal, wenn eine Null gesprochen wird, wird von dem Mikroprozessor 50 ein vier Sekunden währendes Fenstersignal (Fig. 6C) erzeugt.

Drückt nun innerhalb dieses Zeitraums des Fensterimpulses die Testperson den Taster 16, so wird ein Tasterimpuls gemäß Fig. 6D erzeugt. Durch das Zusammenfallen des Tasterimpulses mit dem Fensterimpuls wird - wie oben erläutert - ein Signal erzeugt, welches den Inhalt des Zählers 1 um den Wert 1 erhöht.

Wie etwa in der Mitte in Fig. 6 bei D dargestellt ist, hat die Testperson den Taster 16 betätigt, obschon in dem "relevanten" Kanal 1 überhaupt keine Null gesprochen wurde. Der in der Mitte in Fig. 6D dargestellte Tasterimpuls stellt einen Einfügungsfehler dar, wahrscheinlich verursacht durch die im "nichtrelevanten" Kanal 2 gesprochene Null kurz vorher.

Gemäß Fig. 6A werden zu den Zeitpunkten 12s und 14s jeweils Nullen gesprochen. Beim Zeitpunkt 12s, also mit dem Sprechen der ersten Null wird die Erzeugung eines Fensterimpulses eingeleitet. Dieser Fensterimpuls würde an sich vier Sekunden dauern. Da aber zum Zeitpunkt 14s eine weitere Null gesprochen wird, verlängert sich von diesem Zeitpunkt an das Fenstersignal um weitere vier Sekunden, was insgesamt zu einem sechs Sekunden dauernden Fensterimpuls führt.

Gemäß Fig. 6D sei hier angenommen, daß als Reaktion auf die beiden Nullen zweimal der Taster betätigt wurde, und zwar einmal noch rechtzeitig während des Fensterimpulses, das zweite Mal zu spät außerhalb des Fensterimpulses. Bei dem ersten Tasterimpuls wird der Zähler 1 erhöht, beim zweiten Tasterimpuls wird der Zähler 2 erhöht, der auch bei dem mittleren Tasterimpuls gemäß Fig. 6D erhöht wurde.

Damit hat die Testperson in dem Zeitausschnitt gemäß Fig. 6 insgesamt von den drei Nullen im Kanal 1 zwei Nullen richtig erkannt, so daß der Zähler 1 entsprechend zweimal erhöht wurde. Einmal wurde die Null im Kanal 2 fälschlicherweise dem "relevanten" Kanal zugeordnet. Dies wird ebenso wie das verspätete Betätigen des Tasters im Anschluß an das zweite Fenstersignal als Einfügungsfehler registriert.

Die oben beschriebenen Ausführungsformen lassen sich vielfältig modifizieren. So z. B. lassen sich einige der oben erläuterten und in Fig. 4 schematisch dargestellten Elemente auch durch geeignete Software-Merkmale realisieren.

**Patentansprüche**

1. Gerät zum Testen der Aufmerksamkeit einer Person, **gekennzeichnet** durch folgende Merkmale:
   - aus einem Sprachspeicher (12; 62, 64) werden in gleichmäßigen Zeitabständen Stimuli (Reizworte) ausgelesen;
   - die Stimuli werden mittels eines Sprachgenerators (14; 66, 68) über Lautsprecher oder Kopfhörer (10) für die Testperson hörbar gemacht;
   - ein von der Testperson bei Erkennen eines bestimmten Stimulus-Typs (Target) zu betätigender Taster (16) liefert Tasterimpulse und
   - eine Registriereinrichtung (26; 74, 75, 50, 70) registriert die Tasterimpulse und korreliert diese sowohl mit den Targets als auch mit den von den Targets verschiedenen Stimuli.

2. Gerät nach Anspruch 1,
   dadurch **gekennzeichnet**,
   daß die Stimuli Ziffern sind, von denen z. B. die Ziffer "0" als Target dient.

3. Gerät nach Anspruch 1 oder 2,
   dadurch **gekennzeichnet**,
   daß ein Informationssprachspeicher und/oder eine Anzeige (8) vorgesehen sind als Mittel, der Testperson, die zur Durchführung des Tests benötigten Informationen mitzuteilen.

4. Gerät nach einem der Ansprüche 1 bis 3,
   dadurch **gekennzeichnet**,
   daß der Sprachgenerator (66, 68) mit zwei Kanälen für ein Kopfhörerpaar (10) ausgestattet ist, daß eine Steuereinrichtung (2, 52) eine Umschalteinrichtung aufweist, mit der entweder gleiche Stimuli gleichzeitig auf beiden Kanälen oder verschiedene Stimuli auf den Kanälen erzeugt werden.

5. Gerät nach einem der Ansprüche 1 bis 4,
   **gekennzeichnet**
   durch eine Geschwindigkeits-Umschalteinrichtung (2, 52), mit deren Hilfe eine von mehreren Auslesegeschwindigkeiten der Stimuli auswählbar ist.

6. Gerät nach einem der Ansprüche 1 bis 5,
   dadurch **gekennzeichnet**,
   daß eine Zeitsteuereinrichtung (50, 52) Fenstersignale vorbestimmter Zeitdauer (z. B. 4s, 1,5s) bei jeder Ausgabe eines Targets erzeugt, und daß eine Verknüpfungsschaltung (70, 71 und 72) die Fenstersignale mit den Tasterimpulsen verknüpft.

7. Gerät nach Anspruch 6,
   **gekennzeichnet**
   durch einen ersten Zähler (74), der jeden Tasterimpuls zählt, der mit einem Fenstersignal zusammenfällt.

8. Gerät nach Anspruch 6 und 7,
   **gekennzeichnet**
   durch einen zweiten Zähler (73), der jeden Tasterimpuls zählt, der nicht mit einem Fenstersignal zusammenfällt.

**9.** Gerät nach einem der Ansprüche 1 bis 8,
**gekennzeichnet**
durch eine Rechenschaltung (50), die aus den registrierten Tasterimpulsen einen Fehlerindex ($I_E$) und davon abgeleitete Werte berechnet.

**10.** Gerät nach einem der Ansprüche 1 bis 9,
dadurch **gekennzeichnet**,
daß eine Steuereinheit (2, 50) ein Zeitzählwerk (52) steuert und automatisch eine Reihe von Subtests mit jeweils voneinander verschiedenen Parametern ablaufen läßt.

**11.** Gerät nach Anspruch 10 in Verbindung mit Anspruch 9,
dadurch **gekennzeichnet**,
daß die Rechenschaltung die bei jedem der Subtests registrierten Tasterimpulse einzeln verarbeitet und die Verarbeitungsergebnisse dann miteinander verknüpft.

**12.** Gerät nach einem der Ansprüche 1 bis 11,
dadurch **gekennzeichnet**,
daß der Sprachspeicher ein digitaler Speicher (62) (z. B. RAM) ist, in dem den einzelnen Stimuli entsprechende Sprachsignale unter jeweils einer Adresse oder Adressengruppe gespeichert sind, daß ein Adressenspeicher (58) (z. B. ROM oder RAM) mit seinen Ausgang an den Adreßeingang des Sprachspeichers angeschlossen ist, und daß in dem über ein Adreßregister (56) schrittweise adressierten Adressenspeicher Sprachspeicheradressen auf Pseudo-Zufallsbasis gespeichert sind.

**13.** Gerät nach Anspruch 12,
**gekennzeichnet**
durch einen Taktgeber (54) mit umschaltbarer Taktfrequenz, an welchem das Adreßregister (56) angeschlossen ist.

RAM 4

Reg. 26

Sprach-generator 14

Tasta-tur 5

Steuereinheit 2

Sprach-speicher 12

10

RoM 6

16

Anzeige 8

Fig. 1

20(12)

22

CL

24

T

0 7 1 0 4 5 6 5...

1 3 0 9 8 7 0 2...

Fig. 2

Fig. 3

Fig.4

Fig.5

10

Fig. 6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y<br>A | WO-A-8 500 098 (CLINICAL DIAGNOSTICS, INC.)<br>* Seite 4, Zeile 13 - Seite 7, Zeile 19 *<br>--- | 1,3<br>2,5-8,13 | A61B5/16<br>G09B5/04 |
| Y<br>A | US-A-4 510 942 (R. MIYAMAE ET AL.)<br>* Spalte 7, Zeile 54 - Zeile 63 *<br>* Spalte 10, Zeile 58 - Spalte 11, Zeile 5 *<br>--- | 1,3<br>12 | |
| A | DE-A-2 227 264 (D. CLIFFORD)<br>* Seite 2, Zeile 8 - Seite 5, Zeile 15 *<br>* Ansprüche 1,6,7,10,14 *<br>--- | 1,3,4,9 | |
| A | DE-A-2 830 297 (R. ASSMANN)<br>* Seite 4, Zeile 22 - Seite 5, Zeile 19 *<br>* Ansprüche 1,2; Abbildung 1 *<br>----- | 1,4 | |

**RECHERCHIERTE SACHGEBIETE (Int. Cl.5 )**

A61B
G09B

Der vorliegende Recherchenbericht wurde für alle Patentansprüche erstellt

| Recherchenort | Abschlußdatum der Recherche | Prüfer |
|---|---|---|
| DEN HAAG | 10 JANUAR 1992 | RIEB K.D. |